Europäisches Patentamt

(19) European Patent Office

Office européen des brevets

(11) Publication number: **0 129 361**
**A2**

(12) **EUROPEAN PATENT APPLICATION**

(21) Application number: **84303745.8**

(22) Date of filing: **04.06.84**

(51) Int. Cl.³: **F 16 M 11/12**

(30) Priority: **16.06.83 FI 832199**

(43) Date of publication of application:
**27.12.84 Bulletin 84/52**

(84) Designated Contracting States:
**AT BE CH DE FR GB IT LI NL SE**

(71) Applicant: **Orion-yhtymä Oy**
**Hitsaajankatu 5**
**SF-00810 Helsinki(FI)**

(72) Inventor: **Aarnio, Jaakko**
**Haarniskatie 6 B 13**
**SF-00910 Helsinki(FI)**

(72) Inventor: **Kuronen, Jorma**
**Jorvaksenpuisto A 1**
**SF-02420 Jorvas(FI)**

(74) Representative: **Barlow, Roy James et al,**
**J.A.KEMP & CO. 14, South Square Gray's Inn**
**London WC1R 5EU(GB)**

(54) A supporting arm capable of being turned freely into different positions.

㉗ The specification relates to a supporting arm capable of being turned freely itno different positions, which is made up of at least two straight sections articulated to each other, such a section containing a parallelogram formed by articulated arms in such a way that the ends of the same description of the two long parallel articulated arms (8, 6) of the parallelogram are articulated to a common end piece (2, 12), and, additionally, there being linked to the first articulated arm (8) a tension bar (7) exerting its effect by spring force, the tension bar preventing the parallelogram from collapsing. The object is to provide a supporting arm which has a substantially smooth outer surface and is therefore easy to keep clean and pleasant to handle, and which is furthermore, very sturdy mechanically. In order to achieve this, the second longitudinal articulated arm (6) of the parallelogram is implemented as a tube which surrounds the first, spring-affected articulated arm (8), the articulation points (3, 13) at the ends of the tubular articulated arm (6) preferably being situated outside the center line of the tube. The tubular articulated arm (6) may have an oval cross section in such a way that its sides are substantially flat, the spring-affected articulated arm (8) preferably being situated on the opposite side of the center line of the tube in relation to these articulation points (3, 13).

./...

FIG.1

A supporting arm capable of being turned freely into different positions

The present invention relates to a supporting arm capable of being turned freely into different positions, which is made up of at least two straight sections articulated to each other, at least one of these sections containing a parallelogram formed by articulated arms in such a way that the ends of the same description of the two long parallel articulated arms of the parallelogram are articulated to a common end piece, and, additionally, there being linked to the first articulated arm a tension bar working by spring force, which tends to prevent the parallelogram from collapsing.

Such supporting arms are often used in reading lamps and also, for example, in medicine to hold various treatment instruments in the desired positions. The supporting arm according to the present invention is in particular intended and developed for the latter application, for example, for holding an X-ray tube in a suitable position for intra-oral X-ray photography.

In known supporting-arm structures the articulated arms of the parallelogram are either made up of two clearly separate tubes or profiles, or then one arm is, for example, U-shaped in such a way that the arms can in part overlap. These solutions on the one hand involve the risk that the user's fingers may be caught between the articulated arms, and on the other hand the cleaning of the supporting arm is cumbersome, especially in circumstances where the hygienic requirements are high.

The object of the present invention is to provide a supporting arm which has a substantially conventional mechanism, but in which the above-mentioned disadvantages have been eliminated. In order to achieve this, the supporting arm according to the invention is characterized in that the second longitudinal articulated arm of the parallelogram is implemented as a tube which surrounds the first, spring-affected articulated arm, in which case the articulation points at the ends of this tubular articulated arm are preferably situated outside the center line of the tube. In this way the whole arm is given a smooth, even outer surface, which is very easy to keep clean and which at the same time makes the arm very convenient for the user. In addition, the supporting arm becomes very sturdy mechanically.

The invention is described below in greater detail in the form of an example and with reference to the accompanying draging, in which

Figure 1 depicts the first section of the supporting arm according to the invention, partly cut away  for the sake of illustration,

Figure 2 depicts the articulation points of the base end of the said section, and⸠

Figure 3 depicts schematically how the said first section is linked, by means of an intermediate piece, to the next, substantially similar section.

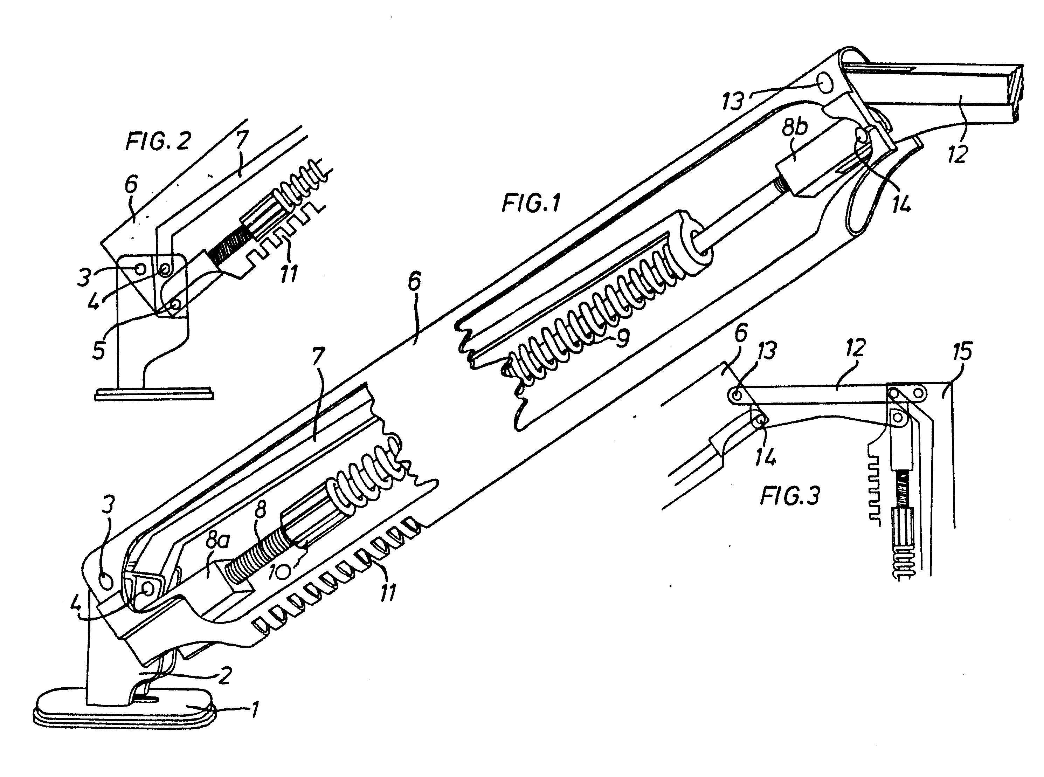

In the drawing, numeral 1 indicates the base of the supporting arm, intended to be secured to a horizontal surface by using screw means or in some other manner known per se. The end piece 2 is secured to the base 1 by means of a vertical pin or the like, not shown, in such a way that the end piece 2 can rotate about the vertical axis in a known manner. The end piece 2 has three articulation points 3, 4, and 5, as is better seen in Figure 2. To articulation point 3 there is articulated a tubular articulated arm 6 having an oval cross section, to point 4 there is articulated a tension bar 7, and to point 5 there is articulated a round bar 8 which is partly threaded.

The tube 6 and the bar 8 are articulated at the upper end to point 13 respectively 14 in a second end piece 12. Both ends of the bar 8 have intermediate pieces 8a, 8b, attached to the bar by means of a thread, the pieces 8a, 8b having articulation points 5 respectively 14 and enabling the length of the bar 8 to be adjusted. Thus is formed in a known manner a parallelogram, the angle points of which are 3, 4, 13 and 14 and which causes, regardless of the angle of the articulated arm 6, the end piece 12 to remain in a horizontal position or in the position into which it has been adjusted by means of the length of the arm 8.

The break spring 9 which has been slipped over the arm 8 and which exerts its effect between the loop at the end of the tension bar 7 and the stop piece 10 which turns on the bar 8 tends to resist the collapsing of the parallelogram and thus compensates for the weight of the arm and the instrument at the end of the arm in such a way that the arm remains in the position into which it is turned. The spring force is adjusted by turning the stop piece 10.

In accordance with the invention, one 6 of the articulated arms is tubular and entirely surrounds the other articulated arm 8 and the other parts of the mechanism. In this manner the supporting arm is completely smooth and pleasant to handle and to keep clean. The tubular articulated arm is naturally also very sturdy mechanically. Even though it does require some material, it is easy and inexpensive to manufacture.

4

To save space, it is advantageous if the tube is oval as shown in the drawing so that its sides are at least in part nearly flat. It is also appropriate to place the articulation points 3 and 13 of the tube 6 clearly outside the center line, the other articulated arm 8 being most suitably situated on the opposite side of the center line in relation to the articulation points. In this manner, ample room is also left for the tension bar 7.

In order to make the control of the spring 9 easy without dismantling the structure, on the curved lower surface of the tube 6 there are successive notches 11 through which the stop piece 10 can be rotated by means of a suitable tool. When necessary, it is of course also possible to arrange a possibility for controlling the length of the articulated arm 8 itself in this manner.

Figure 3 shows the principle of securing the two sections of the supporting arm to each other by means of the end piece 12. The continuation section is in this case similar to the section already described, and numeral 15 indicates a tubular articulated arm similar to that indicated by 6.

5

Claims

1.   A supporting arm capable of being turned freely into different positions, which is made up of at least two straight sections articulated to each other, at least one of these sections containing a parallelogram formed by articulated arms in such a manner that the ends of the same description of the two long parallel articulated arms (8, 6) of the parallelogram are articulated to a common end piece (2, 12), and, additionally, there being linked to the first articulated arm (8) a tension bar (7) exerting effect by spring force, the tension bar (7) tending to prevent the parallelogram from collapsing, c h a r a c t e r i z e d i n  that the second longitudinal articulated arm (6) of the parallelogram is implemented as a tube surrounding the first, spring-affected articulated arm (8), the articulation points (3, 13) at the ends of this tubular articulated arm (6) preferably being situated outside the center line of the tube.

2.   A supporting arm according to Claim 1, c h a r a c t e r i z e d   i n   that the tubular articulated arm (6) has an oval cross section, its sides being substantially flat, and that the articulated arm (8) under the effect of the spring (9) is located on the opposite side of the center line of the tube in relation to the articulation  points (3, 13) of the tubular articulated arm (6).

3.   A supporting arm according to Claim 1 or 2,  in which the spring (9) is a helical spring around the first, inner articulated arm (8), the spring resting against a stop piece (10) adjustable in the longitudinal direction of the articulated arm (8) by means of, for example, a thread, c h a r a c t e r i z e d   i n   that in the area of the stop piece (10) there are one or several openings (11) in the second articulated arm (6) in order to enable the stop piece (10) to be adjusted through the opening by means of a suitable tool.

4. A supporting arm according to Claim 3, characterized in that the openings consist of successive notches (11) made on the lower surface of the articulated arm (6).

5. A supporting arm according to any of the above claims, characterized in that the articulated arm (8) under the effect of the spring (9) is secured to the end piece (2; 12) by means of an intermediate piece (8a; 8b), which is attached to the articulated arm by means of a thread in order to control the length of the articulated arm (8).

FIG. 2

FIG.1

FIG.3

1/1

0129361